# EUROPEAN PATENT APPLICATION

(11) **EP 1 422 233 A1**
(43) Date of publication of application: **26.05.2004**
(21) Application number: 02025940.4
(22) Date of filing: 20.11.2002
(51) Int. Cl.: C07H 21/00

(54) **Chemical copying of connectivity information in branched DNA structures**

(71) Applicant: Von Kiedrowski, Günter, 44797 Bochum-Stiepel (DE); Ruhr-Universität Bochum, 44780 Bochum (DE)
(72) Inventor: Kiedrowski, Günter von, 44797 Bochum-Stiepel (DE); Eckardt, Lars Henning, 44799 Bochum (DE); Naumann, Kai, 44803 Bochum (DE); Pankau, Wolf Matthias, 44801 Bochum (DE); Rein, Michael, 51427 Bergisch Gladbach (DE)
(74) Representative: Helbing, Jörg, Dr. Dipl.-Chem.

(57) **Abstract**

The present invention relates to a method for chemical copying of connectivity in branched DNA structures. Further, the present invention relates to a kit for carrying out said method, and the building block and the template as well as the use thereof.

## Description

The present invention relates to a method for chemical copying of connectivity in branched DNA structures. Further, the present invention relates to a kit for carrying out said method, and the building block and the template as well as the use thereof.

### Field of the Invention

DNA-nanobiotechnology is developing to become a highly active area of interdisciplinary research whose scope is continuously expanding and whose limitations are still not settled (Seeman, N.C., Synlett, 1536-1548 (2000); Trends Biotechnol. 17: 437-443 (1999); Angew. Chem. 110: 3408-3428 (1998); Angew. Chem. Int. Ed. Engl. 37: 3220-3238 (1998); Acc. Chem. Res. 30: 357-363 (1997)). Examples include the construction of DNA nanostructures from linear oligonucleotides that self-assemble into 3- and 4-way junctions, which can be covalently ligated to produce objects ranging from cubes to 2D-crystals (Chen, J., Seeman, N.C., Nature 350: 631-633 (1991); Zhang, Y., Seeman, N.C., J. Am. Chem. Soc. 116: 1661-1669 (1994); Mao, C., Sun, W., Seeman, N.C., Nature 386: 137-138 (1997); Winfree, E., Liu, F., Wenzler, L. A., Seeman, N.C., Nature: 394, 539-544 (1998)), the assembly of nanometer-sized macrocycles from complementary V-shaped molecules in which the 3'-ends of two oligonucleotides were attached to a bifunctional linker (Shi, J., Bergstrom, D.E., Angew. Chem. 109: 70-72 (1997); Angew Chem. Int. Ed. Engl. 36: 111-113 (1997)), the utilization of dendrimeric oligonucleotides for strong and cooperative binding to immobilized complementary strands (Shchepinov, M.S. et al., Nucleic Acids Res. 25: 4447-4454 (1997); Shchepinov, M.S. et al., Nucleic Acids Res. 27: 3035-41 (1999)), the formation of DNA-protein networks between 5'-biotinylated oligonucleotides and streptavidin (Niemeyer, C.M. et al., Nucl. Acids Res. 22: 5530-5539 (1994); Niemeyer, C.M. et al., Angew. Chem. 112: 3183-3187 (2000); Angew. Chem. Int. Ed. Engl. 39: 3055-3059 (2000); Niemeyer, C.M., Angew. Chem. 113: 4254-4287 (2001); Angew. Chem. Int. Ed. Engl. 40: 4128-4158 (2001)), the generation of ordered nanocrystals in which double stranded oligonucleotides define the distance between metal clusters and nanoparticles (Mirkin, C. A. et al., Nature 382: 607-609 (1996); Elghanian, R., Science 277: 1078-1080 (1997); Storhoff, J. J., Mirkin, C. A., Chemical Reviews 99: 1849 (1999); Taton, T.A. et al., Science 289: 1757-1760 (2000); Park, S.-J. et al., Science 295: 1503-1506 (2002)), the spatial positioning of metal clusters and other functional elements using metal cluster-oligonucleotide monoconjugates and complementary DNA templates (Alivisatos, A. P. et al., Nature 382: 609-611 (1996); Loweth, C. J. et al., Angew. Chem. 111: 1925-1929 (1999); Angew. Chem. Int. Ed. Engl. 38: 1808-1812 (1999)), the usage of these monoconjugates as fluorescence quenchers (Dubertret, B. et al., Nat. Biotechnol. 19: 365-370 (2001)) and single-molecule radio frequency receivers (Hamad-Schifferli, K., Nature 415: 153-155 (2002)), the utilisation of DNA as well as DNA scaffolds for logical devices (Mao, C. et al., Nature 397: 144 (1999); Mao, C. Nature 407: 493 (2000); Yan, H. et al., Nature 415: 62-65 (2002)), molecular machines (Yurke, B. et al., Nature 406: 605-608 (2000), and for molecular electronics (Braun, E. et al., Nature 391: 775 (1998); Kelley, S.A., Barton, J. K., Science 283: 375 (1999); Bixon, M. Proc. Natl. Acad. Sci. U.S.A. 96: 11713 (1999); Giese, B. Acc. Chem. Res. 33: 631 (2000)), nanowiring (Adleman, L. Science 266: 1021-1024 (1994); McCaskill, J., Niemann, U. DNA Computing 103-116 (2000)) and new approaches in parallel computation addressing highly combinatorial problems (Scheffler, M. et al., Angew. Chem. 111: 3313-3316 (1999); Angew. Chem. Int. Ed. Engl. 38: 3312-3315 (1999)). We have recently reported on the noncovalent synthesis of nanoobjects from symmetrical 3'-trisoligonucleotidyls, viz.Y-shaped molecules in which the 3'-ends of three identical sequences are held together by a trislinker (Scheffler, M. et al., Angew. Chem. 111: 3313-3316 (1999); Angew. Chem. Int. Ed. Engl. 38: 3312-3315 (1999); Jordan, S., thesis, University of Göttingen (1993); Scheffler, M., thesis, Ruhr-University, Bochum (1999)). It was shown that kinetic control during self-assembly lead to the formation of small aggregates, whose topologies can be formally described with hydrocarbon nomenclature if one understands a DNA double strand as a C-C bond. Objects having the topology of acetylene, cyclobutadiene, and isobutane could be identified (Scheffler, M. et al., Angew. Chem. 111: 3313-3316 (1999); Angew. Chem. Int. Ed. Engl. 38: 3312-3315 (1999)).

The fields of chemical self-replication and information-based nanotechnology were anticipated by early visions (von Neumann, J. University of Illinois Press, Urbana (1966); Todd, A., Interscience, New York, p. 245 (1956); Feynman, R.P., Miniaturization, 282-296 (1961)) came both into experimental reality with DNA as "material" (Kallenbach, N.R. et al., Nature 305: 829-831 (1983); Chen, J., Seeman, N.C., Nature 350: 631-633 (1991); von Kiedrowski, G. Angew. Chem. Intl. Ed. Engl. 25: 932-935 (1986)) spread to conquer other classes of molecular systems (Zielinski W.S., Orgel, L.E., Nature 327: 346-347 (1987); Tjivikua, T. et al., J. Am. Chem. Soc. 112: 1249-1250 (1990); Lee, D.H. et al., Nature 382: 525-528 (1996)) began to flourish in various applications (Adleman, L., Science 266: 1021-1024 (1994); Mirkin, C.A. et al., Nature 382: 607-609 (1996); Alivisatos, A.P. et al., Nature 382: 609-611 (1996); Braun, E. et al., Nature 391: 775-778 (1998); Winfree, E. et al., Nature 394: 539-544 (1998); Yurke, B. et al., Nature 406: 605-508 (2000); Niemeyer, C.M., Angew. Chem. Int. Ed. 40: 4128-4158 (2001)) and are now ready to merge. The link between these fields was foreseen to lead to artificial self-replicating molecular machinery on the nanometer scale (Drexler, K.E., Anchor press, Doubleday (1986); Winkless, N., Browning, E. Rotics press, Portland (1978)), called nanobots, whose underlying concept was criticized from chemical and physical reasoning (Smalley, R.E., Scientific American 285: 76-77 (2001)). However, if one considers nanobots as threedimensionally engineered noncovalent nanoscaffolds - arraying modular functions used as catalytic or binding machinery - that can be instructed to replicate by remote control, many pieces of technology needed for their implementation became recently available. Gold cluster-labelled molecules were remotely controlled by radio frequency causing local and selective inductive heating (Hamad-Schifferli et al., Nature 415: 152-155 (2002)). Charged molecules were electrophoretically steered and manipulated on the surface of an electronic chip (Kricka, L.J., Nature Biotechn. 16: 513-514 (1998). Surface-promoted replication and exponential amplification of DNA analogues (Luther, A. et al., Nature 396: 245-248 (1998)) may find particular applications for the cloning and copying of informational nanostructures on the surface of such chips.
As Seeman et al. have demonstrated with several examples, the construction of "threedimensional" DNA nanoobjects such as cubes (Kallenbach, N.R. et al., Nature 305: 829-831 (1983)) requires junction elements with at least three arms. Seeman's strategy involved noncovalent junctions between assemblies of three or more linear oligonucleotides whose sticky ends allowed covalent connection by ligation. We have mentioned that the reverse strategy based on covalent junction elements and noncovalent assembly of the nanostructures offers a potential for nanostructure replication (Scheffler, M. et al., Angew. Chem. Int. Ed. 38: 3311-3315 (1999)). In the meantime, an example was reported, in which a linear template directs the synthesis of a branched product having two strands in parallel orientation (a [2+1]-arm bisoligonucleotidyl in our notation) (Fujimoto, K. et al., Tetrahedron Lett. 41: 9437-9440 (2000)). We moreover described that functionalized DNA nanoscaffolds with stiff tensegrity such as tetrahedra self-assemble from maximally instructed sets of 3- or [3+1]-arm 3'-linked trisoligonucleotidyl junctions (Dorenbeck, A. et al., thesis Ruhr Univ. Bochum (2000)).

Additional supramolecular nanosystems based on pRNA (pyranosyl-RNA) monomers were disclosed by A. Eschenmoser, N. Windhab et al. in DE-A-19741738, DE-A-19741739, DE-A-19741715, DE-A-19837387, WO 98/25943, Helv. Chim. Acta 76, 2161 (1995), Helv. Chim. Acta 78, 1621 (1995), Angew. Chem 108, 1619-1623 (1996) and Synlett 940-944 (1999).

However, hitherto there is no mention that non-linear templates can be copied into a branched product and thus enable synthetic copying of a branched junction as an information stored in the structure of the template.

### Summary of the Invention

Accordingly, the method of the present invention differs from the latter example mentioned above in that the connectivity information in a branched junction is copied. The prime application for this mode of copying is conceived in the replication of instructed 3-dimensional nanostructures and functionalized nanoscaffolds from sets of linear oligonucleotides. Surprisingly, it was found that the connectivity information present within the templates of the three-dimensional structures referred to above. Such copying is based on template-directed linkage of branched templates employing robust and efficient coupling reactions such as hydrazone formation chemistry.
The present invention thus provides
(1) a method for the chemical copying of connectivity which comprises coupling a set of linear oligonucleotide segments to a junction element in the presence of one or more branched templates having oligonucleotide arms to form branched copies of the templates
(2) a kit for chemical copying of connectivity comprising one or more of the compounds as defined in (1) above, and
(3) the copied template as defined in (1) above.

### Description of the Figures

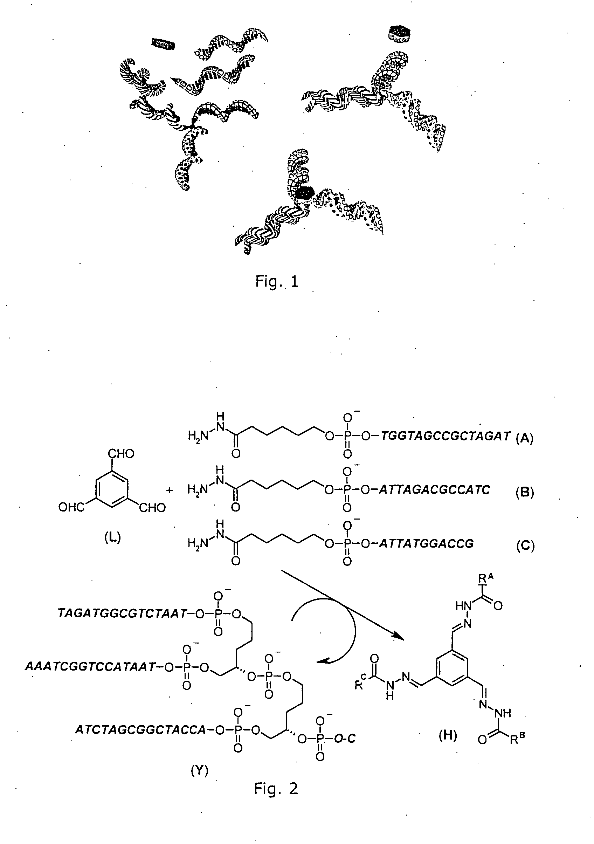

Figure 1. Illustration of chemical connectivity copying (CCC) by template-directed trislinking. Left: The copying process involves a 3'-trisoligonucleotidyl with three individually defined sequences, three linear complements whose 5'-ends are drawn as blunt ends, and a trislinker shown as a honeycomb cap. Right: Hybridisation leads to a quartermolecular complex in which the blunt ends come into close spatial proximity. Bottom: The trislinker has reacted to connect the 5'-ends of the copy.
Figure 2. Trislinking of 5'-hydrazide-modified oligonucleotides A, B, C with trislinker L in the presence of 3'-trisoligonucleotidyl template Y yields trishydrazone H in which R^{A}, R^{B}, R^{C} corresponds to A, B, C, respectively. Sequences are always notated in the 5' → 3'-direction.

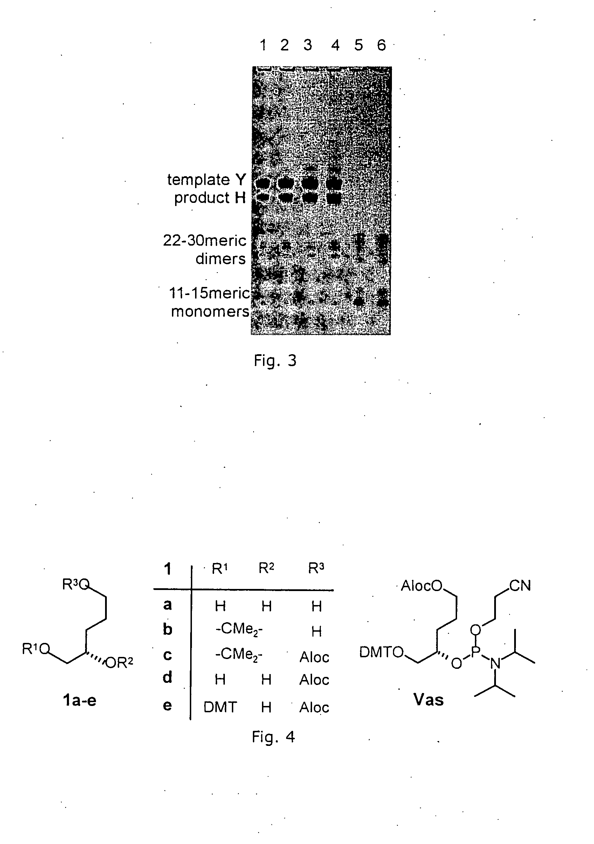

Figure 3. Denaturating PAGE of a nearly complete template-directed trislinking reaction after 2h incubation at room temperature; lanes 5, 6: no template. In lanes 1, 3, 5 one equivalent of trislinker L was added; other lanes: 2.5 eq. of L. Lanes 3-6: trislinking in the presence of 100 eq. NaCNBH₃. Trislinking (in lane 1) was initiated by adding 1 µl of a 500 µM solution of L in dimethylformamide to 10 µl of a solution containing 10 µM of each A, B, C, Y in 100 mM MOPS-buffer (Na⁺/H⁺-form) at pH 5.6, vortexing, heating (2 min at 60 °C) and rapid cooling to room temperature. The gel (10% acrylamide, 7 M urea) was stained for 10 min with SYBR® Gold nucleic acid gel stain (10000X in DMSO, Molecular Probes).
Figure 4. Synthesis of linker Vas; 1a→1b) p-TosOH, acetone; 1b→1c) Allyloxycarbonylchloride, pyridine/THF; 1c→1d) 1N HCl/THF 1:1(v/v) 1d→1e) DMT-Cl, pyridine; 1e→Vas) 2-(cyanoethoxy)-bis-N,N-(diisopropylamino)-phosphane, dichloromethane.

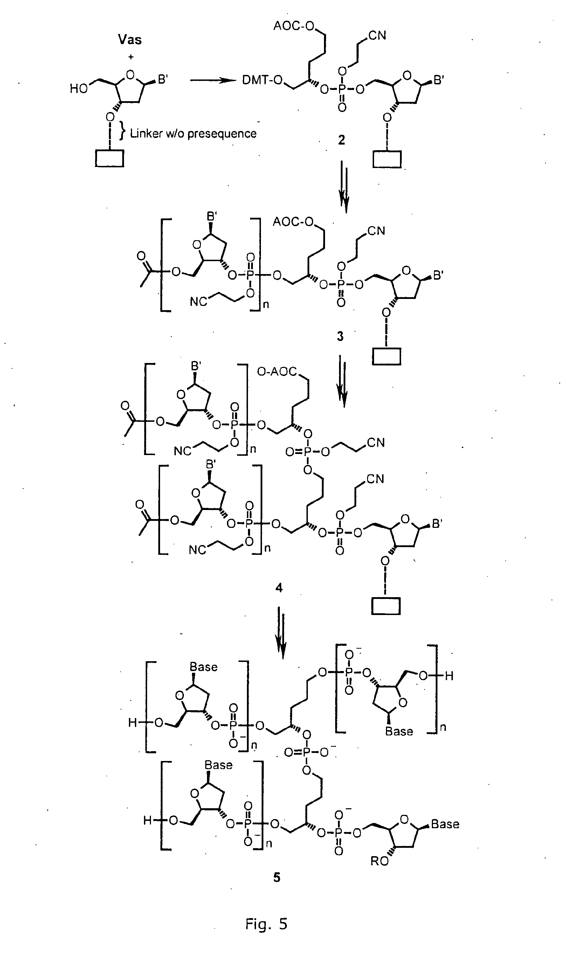

Figure 5. Synthesis of 3'-trisoligonucleotidyls 5. R = H for 3-arm units with C as leader base, R = presequence for 3+1-arm units. Standard phosphoramidite protocol for a Pharmacia Gene Assembler synthesis on 500 A CPG, 1.3 µmol scale, with the following modifications with respect to amidite replenishment and coupling times: Vas: 2 x 15 min (first), 3 x 15 min (second). Nucleoside amidites: 2 x 2 min (first strand), 3 x 2 min (second strand), 3 x 15 min (first amidite third strand), 3 x 2 min (following amidites). Allyloxycarbonyl deprotection: 2 x 15 min of a solution containing 4.4 mM bis(dibenzylidenacetone)palladium(0), 4.4 mM bis-1,2-diphenylphosphanylethane, 13.2 mM pyrrolidine in acetonitrile at a flow rate of 0.5 ml/min. Final deprotection with conc. NH₄OH at 55°C for 12 h in a pressure-tight tube. Cf. Table 1a,b for further details.

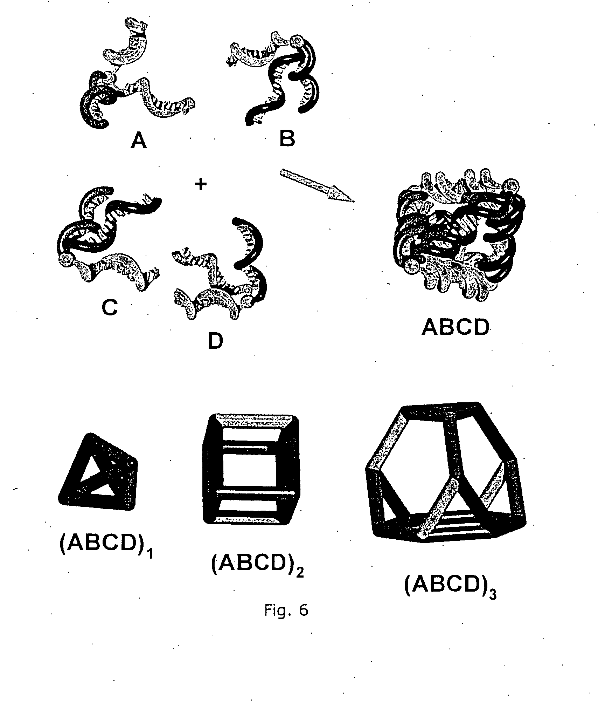

Figure 6. Illustration of the noncovalent synthesis; the tetrahedral nanoobject is the smallest member in a series of spherically closed superstructures. The next higher members have the topologies of cubane and diademane.

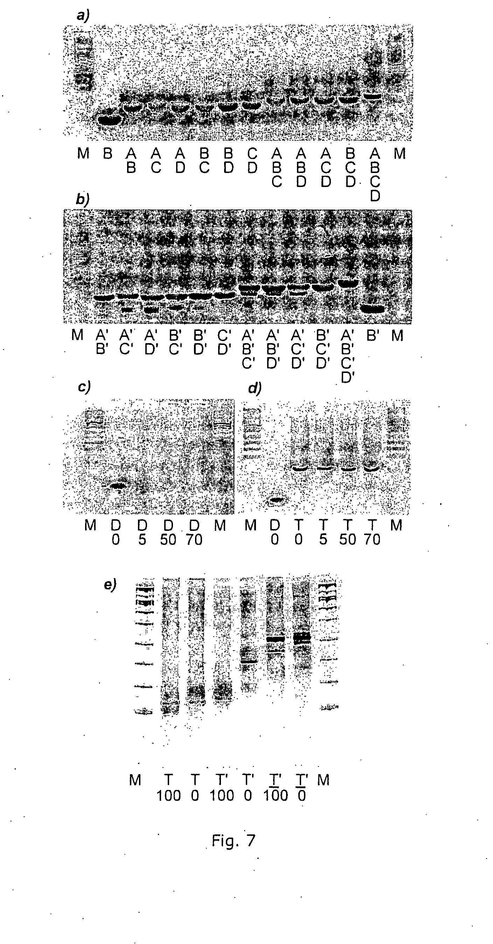

Figure 7. Native gel electrophoresis experiments on the formation and digestion of tetrahedral nanoobjects; a) complexes from trisoligos without presequence; b) complexes from trisoligos with presequence; c) digestion of trisoligo D with mung bean nuclease for the given number of hours at T = 20 °C; c),digestion of T (=ABCD); d) digestion of T, T' (= A'B'C'D') and T' (= T' with saturated presequences). M denotes the marker pUC mix marker 8 (MBI Fermentas). In all experiments the sum of trisoligo concentrations was 5 µM in 100 mM NaCl, 10 mM Na₂HPO₄, pH 8.6. The samples were denatured at T = 95 °C and cooled to 50 °C with an slope of 0.1 °C s⁻¹ and from 50 °C to 0 °C with 0.1 °C s⁻¹. Electrophoresis in (a-d) was performed on an 3 % agarose gel (SeaKeam LE; FMC) with 85 V for 3 h using an ice-bath for cooling. The gels were stained with Gelstar (Biozym). Electrophoresis in (e) took place on a 6 % polyacrylamide gel applying 100 V for 2.5 h, ice bath cooling, and staining with SYBRGold™ (Molecular Probes). All digestion experiments were carried out at 20 °C and initiated by the addition of 0.5 µl of the enzyme solution (50 u/µl) to 10 µl of the respective trisoligo solution.

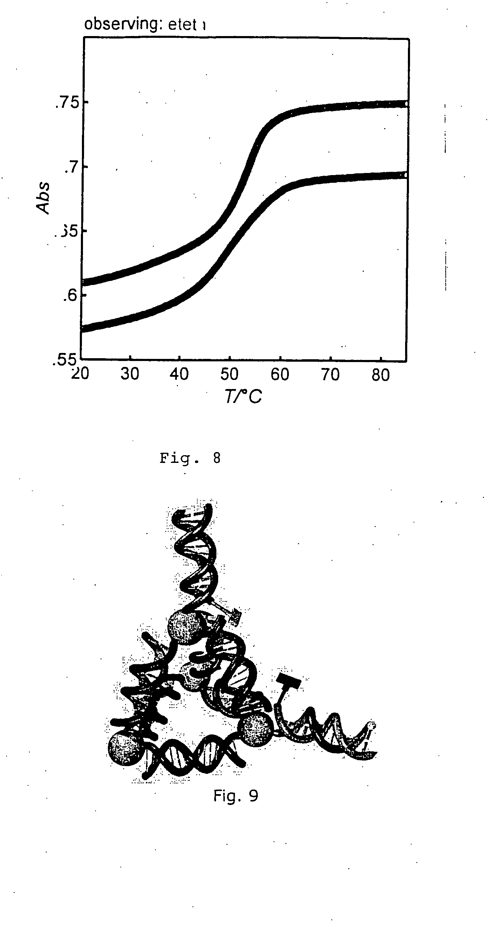

Figure 8. UV-melting curves of a mixture of linear oligos (Table 1b, Nr. 1-12, lower curve) and a mixture of the trisoligos A, B, C, D (upper curve). Conditions: 273 nM of each oligo component, 100 mM NaCl, 10 mM Na₂HPO₄, pH 8.6, heating rate at 0.5 K min⁻¹, λ = 260 nm.
Figure 9. Illustration of a tetrahedral nanoscaffold composed of two 3-arm- and two [3+1]-arm building blocks. The presequences are paired with linear complements bearing fluorescent dyes as 3'-modifications. Linkers are symbolized as balls, dyes as rectangular blocks.

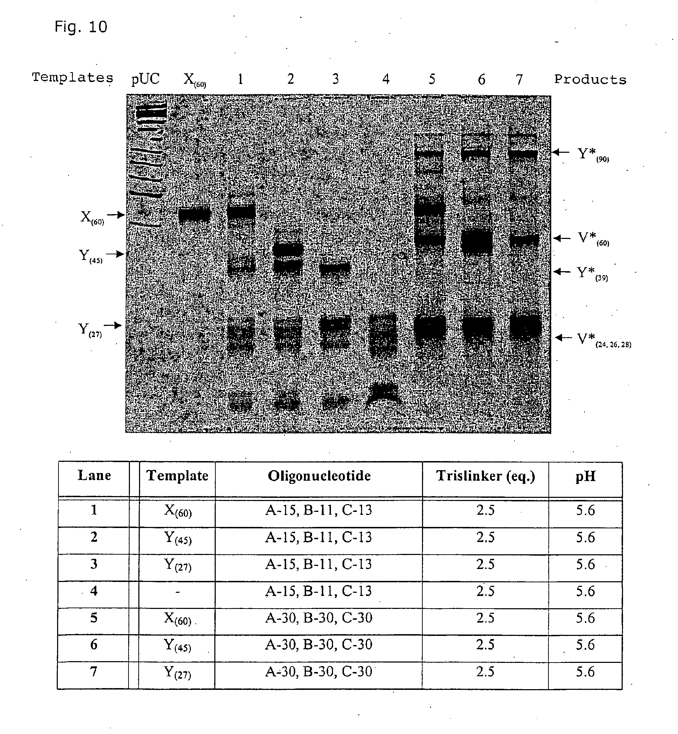

Figure 10. Trislinking of 5'-hydrazide-modified oligonucleotides with extended strands in presence of different templates. The pointers at the right hand side of the gel designate the products, those at the left hand side designate the templates, Y* representing trislinked speicies and V* representing bislinked species.

### Detailed Description of the Invention

The general procedure of chemical connectivity copying (CCC) is outlined in Figure 1. Using a previously described (Dorenbeck, A. et al., thesis Univ. Bochum (2002)) asymmetric tris-15-mer as the template, we investigated a number of conceivable linking chemistries based on linear oligonucleotides equipped with a reactive group at their 5'-ends and a suitable trislinker (Figure 1 outlines the general procedure). Native ligation techniques (Dawson, P.E. et al., J. Am. Chem. Soc. 119: 4325-4329 (1997)) - using a 5'-cysteinyl-modifier similar to the one reported (Stetsenko, D.A., Gait, M.J., J. Org. Chem. 65: 4900-4908 (2000)) and a water soluble tris-thioester designed by us - as well as thiol-maleimide additions employing a 5'-thiol-modifier and a tris-maleimide (Cochran, J.R., Stern, L.J., Chemistry & Biology 7: 683-696 (2000)) both failed in our hands to give considerable yields of the tris-copies due to hydrolysis reactions. Fast and robust trislinking could however be achieved by the hydrazone formation chemistry (Paddatz S. et al., Nucl. Acids Res. 30, 4793-4802 (2002)) shown in Figure 2.

In a preferred embodiment of the invention
(i) the molecular templates and the copies of the templates have at least three, preferably linear oligonucleotide arms/segments; and/or
(ii) said oligonucleotide segments and said oligonucleotide arms independently comprise from 3 to 1000, preferably from 5 to 100, most preferably from 9 to 30 nucleotides; and/or
(iii) the linear oligonucleotide segments of the copies of the templates have at least 3 consecutive nucleotides hybridising to at least 3 consecutive complementary nucleotides of one of the oligonucleotide arms of the template; and/or
(iv) the oligonucleotide arms of the templates have the same or different nucleotide sequence, preferably have different nucleotide sequences, and preferably are covalently bound to a core structural element; and/or
(v) the linear oligonucleotide segments of the copies of the templates are covalently coupled to a junction element having at least three functional groups which are reactive to a corresponding functional group present on each of said linear oligonucleotide segments; and/or
(vi) the junction element is capable of making covalent bonds to at least 3 oligonucleotide segments, preferably the junction element has at least three functional groups as defined under (v) above.

It is furthermore preferred that the templates have the formula (I)

L-D(S-A)ₙ (I)

wherein D is the core structural element, A represents independently the oligonucleotide arms of the template which may be independently oriented in both possible directions, S is a suitable spacer group capable of connecting the oligonucleotides arms to the core, L is an optional linker suitable for binding the template to a solid support, n is ≥ 3, preferably D is selected from a substituted or unsubstituted branched alkyl residue, a cycloalkyl residue, an aryl residue, a phosphanyl or phosphonate residue, a phosphate, an amine, a cyclopeptidyl residue and a cyclodextryl residue, S and L are functionalized linear alkyl residues which may be substituted or may carry heteroatoms and may preferably contain phosphates as functional moieties, and n is from 3 to 5, preferably is 3.

Particularly preferred templates correspond to formula (II) 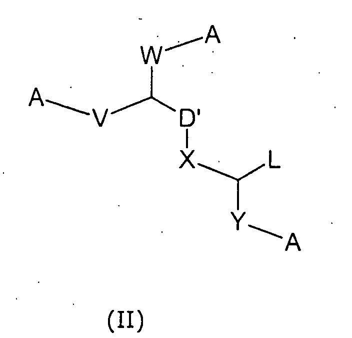 wherein A and L are as defined above, D' has the same meaning as D defined above, and V, W, X and Y are optionally substituted alkyl residues; or the template correspond to formula (III) 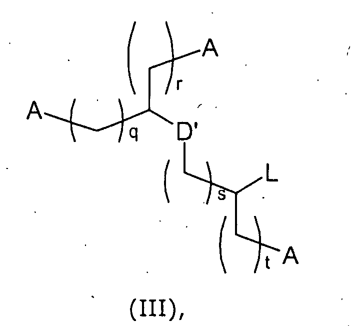 wherein A and L are as defined above; D' is a phosphate, q, r, s and t are integers each ranging independently from 0 to 20, provided that r and q are not 0 at the same time, preferably q, r, s and t range from 1 to 5, more preferably q = t = 1 and r = s = 3.

In a further preferred embodiment the linear oligonucleotide segments A' comprise optionally a suitable spacer group S conjugated to one of its ends or to a central portion of the segment, which are bonded to a functional junction element B' so that the resulting copies of the templates correspond to the formula (IV)

B(S-A')ₙ (IV)

wherein S is a suitable spacer group as defined in claim 3, A' has the same meaning as A defined in claims 3 or 4, n has the same meaning as defined in claims 3 or 4, and B is a junction element as defined in claim 2.

It is preferred that the variants of the above formula (IV)
(i) B is cyclic or heterocyclic, preferably an aromatic or heteroaromatic ring having at least 3 same or different functional groups, more preferably the ring having 3 identical functional groups, in C_{3V} symmetry said functional groups being capable of bounding to the linear oligonucleotide segments or their conjugates of formula (I) and/or (II), still more preferably being selected from one or more of -OH, -SH, -CHO, -COOH, -NH₂, -NHNH₂, ONH₂ maleimide, or vinylsulfone and functionally equivalent groups, still more preferably being an electrophilic group, most preferably being -CHO; and/or
(ii) S is a linear alkyl residue connected to A' via phosphodiester linkage and having from 1 to 20, preferably from 1 to 6 carbon atoms, being optionally interrupted by a heteroatom and having one or more functional groups independently selected from -OH, -SH, -CHO, -COOH, -NH₂, -NHNH₂, at least one of which is reactive to a functional group of the junction element as defined under (i) above, preferably is a nucleophilic, nitrogen containing group, more preferably is -NHNH₂.

Particularly preferred is that
(i) S corresponds to formula (V) 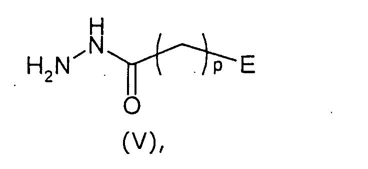 wherein
   (a) p is an integer ranging from 1 to 10, preferably from 1 to 6, most preferably is 5; and
   (b) E is a chemical bond, a heteroatom, a phosphate, a pyrophosphate, an amide or carbonyl bond to A'; and/or
(ii) B corresponds to formula (VI) and (VII):

   N[(CH₂)₂(CH₂)ₖNHCOCHO]₃ (VI) 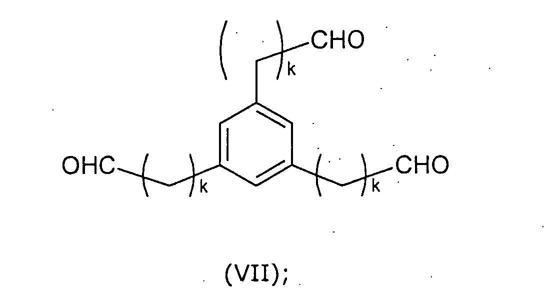
wherein k is from 0 to 2, preferably k is 0.

Suitable oligonucleotides segments and arms for the method of the invention are DNA, RNA, PNA (peptide nucleic acids; Letsinger, R. L., et al. Nature 382, 607-609 (1996) and Schultz, P. G., et al., Nature 382, 609-611 (1996)), LNA (locked nucleic acids), morpholino-nucleotides (Stirchak E. P., et al., Nucl. Acids Res. 17(15), 6129-41 (1989), WO 00/50626 and U.S. Patent No. 5,831,014), TNA (Threofuranosyl Oligonucleotides; Wu, X., et al., Organic Letters 4(8), 1283-1286 and 1279-1282 (2002)), CNA (δ-peptide analogues of pyranosyl RNA, cyclohexylnucleooligoamides; Quinkert, G. et al., Helv. Chim. Acta 83(6), 1049-1078 (2000), WO 99/15509 and WO 97/43232) based structures and pRNA (pyranosyl RNA polymers of Eschenmoser and Windhab referred to above). Most preferred are DNA structures and pRNA polymers. Particularly preferred is the system shown in Fig. 2.

Three 5'-hydrazide-modified oligonucleotides, a 15-mer (A), a 13-mer (B) and a 11-mer (C), complementary to the 3'-ends of the tris-15-meric template (Y) were synthesized employing standard solid-phase protocols. The 5'-hydrazide moieties were introduced using a modifier amidite linkage moiety (Raddatz, S., et al., Nucl. Acid Res., 30:4793-4802 (2002)). For a statistical, non-template-directed synthesis we expect ten 5'-trislinked species, ranging from a branched 33-mer to a branched 45-mer as well as six bislinked species between 22-mers and 30-mers. For a template-directed synthesis we expect a single branched 39-mer as well as 3 of the six possible bislinked species, if the reaction has not reached completion. Figure 3 shows that in the presence of the template the expected 39-mer is formed as the major product, while in the absence of the template there are no trislinked products at all within the limits of delectability of a PAGE analysis. There is also evidence that the template directs the formation of the intermediate bislinked species, whose distribution clearly differs from the one observed for the statistical synthesis. Reductive amination conditions using sodium cyanoborohydride as reported for other imine-based template-directed ligation reactions (Li, X. et al., J. Am. Chem. Soc. 124: 746-747 (2002)) were tested but found to be not necessary for obtaining chemical stability of the copies. We will report on the detailed kinetic analysis and a further optimisation of the connectivity copying reaction later. From the existing data we allow ourselves to conclude that, in spite of its age, hydrazone formation is fast and robust aqueous organic chemistry that may compete with other efficient chemical ligation reactions recently developed for the copying of linear templates (Li, X. et al., J. Am. Chem. Soc. 124: 746-747 (2002); Sando, S., Kool, E.T., J. Am. Chem. Soc. 124: 2096-2097 (2002); Gartner, Z.J., Liu, D.R., J. Am. Chem. Soc. 123: 6961-6963 (2001)).

Alternatively, the corresponding pRNA structures can be utilized. Said pRNA allows the use of the so-called "I-linker", a tryptamine based pyranoyl nucleoside, which not only allows linkage without a spacer group but also allows linkage in the middle of a nucleotide strand.

While one end of the linear construction elements is required for the trislinking, the other may be conjugated with modular functions either to be presented by the target nanoscaffolds, or to be utilized in the course of nanoscaffold replication. The SPREAD-procedure (Luther, A. et al., Nature 396: 245-249 (1998)), for example, would require that one of the three available ends per junction is reserved for immobilisation, while the other two can be conjugated with other molecules. A tetrahedral nanoscaffold could thus be loaded with 8 different functional modules whose 3-dimensional positioning is encoded in the connectivity of the trisoligonucleotidyl junctions. Although the second step for a SPREAD-type replication, namely the copying of 5'- into 3'-trisoligonucleotidyls waits for its elaboration, there are no principal reasons, why a trislinking using the same or a similar chemistry could not be carried out here. The same is due for the copying of connectivity information in branched versions of other classes of informational molecules. Particularly Eschenmoser's pRNA (Eschenmoser, A.,

Science 284: 2118-2124 (1999)) molecules are prime candidates for such new approaches in nanotechnology, as their backbone can be conceived by a walk through the diamond lattice, giving exceptional backbone rigidity. These molecules do not form double helices but more or less linear ladder-type assemblies whose linear scalability offers great promises for nanoscaffold engineering. We thus consider it as advantageous that the copying of connectivity information is a purely chemical process. On the DNA side however, the asymmetric 5'-trisoligonucleotidyls reported here carry free 3'-ends which may be extended using a polymerase and may thus lead to a PCR-based technology for the amplification of geometrically defined DNA nanostructures. For example, a tetrahedral nanostructure may be amplifiable if one starts from a set of four 5'-trisoligonucleotidyls each equipped with three individually defined sequences to give a total of 12 modular primers. The connectivity information for the scaffold may be seeded by tiny amounts of six linear and non-interacting sequences encoding the links between the nodes of the tetrahedron. Once an amplification of nano-scaffolded arrays of functional modules becomes available, either based on PCR, SPREAD or another procedure, in vitro evolution techniques similar to SELEX (Tuerk, C., Gold, L., Science 249: 505-510 (1990); Ellington, A.D., Szostak, J.W., Nature 346: 818-822 (1990); Robertson, D.L., Joice, G.F., Nature 344: 467-468 (1990)) may be employed to find an optimal array of functions for the given task. Among the possible tasks for such constructs are catalysis, binding, and sensing tasks, the arraying of inorganic nanoscale materials, and possibly the evolutionary design of nanoelectronic or photonic "circuitry". As such, nano-bots - being safe in the sense that their replication cannot proceed autonomously but needs remote control and a technical microenvironment - may be no longer a matter of technological dreams, visions and fears but something useful whose implementation waits on the doorstep.

The trisoligonucleotidyls with 3 different sequences which can be untilized for noncovalent synthesis of defined nanoobjects are disclosed in Scheffler, M., thesis, Ruhr-University, Bochum (1999); Dorenbeck, A., thesis, Ruhr-University, Bochum (2000). For the construction of these building blocks, a bifunctional linker amidite Vas (for other branching amidites and their applications see: Horn, T., Urdea, M.S., Nucleic Acids Res. 17: 6959 (1989); Horn, T. et al., Nucleosides & Nucleotides 8: 875 (1989); Horn, R. et al., Nucleic Acids Res. 25: 4842 (1997)) was synthesized in enantiomerically pure form (Figure 4) (Scheffler, M., thesis, Ruhr-University, Bochum (1999); Dorenbeck, A., thesis, Ruhr-University, Bochum (2000)). Triol (1a; the following compound designators in brackets refer to the corresponding compounds shown in Figures 4 and 5) is readily available from L-glutamic acid on a multigram scale by a two-step procedure involving diazotation, lactonisation (Herdeis, C., Synthesis, 232 (1986)) and subsequent exhaustive reduction with BMS (Hornemann, A. M., Lundt, I., Tetrahedron Lett., 53: 6879 (1997). Regioselective introduction of the allyloxycarbonyl (Aoc) group following published protocols (Angyal, S. J., Beveridge, R. J., Carbohydr. Res., 65: 229 (1978)) yielded diol (1d) (via (1b) and (1c)), which was monoprotected with 4,4'-dimethoxytrityl chloride in pyridine (86%) furnishing (1e) and subsequently phosphitylated to give (Vas) in 89% yield (experimental details in Example 1). To confirm its enantiopurity and proper configuration, alcohol (1d) was converted into its Mosher ester and analyzed by ¹⁹F NMR spectroscopy (experimental details in Example 2).

Figure 5 depicts how we synthesized the target 3'-trisoligonucleotides (3-arm-units) including those with a 5'-presequence (3+1-arm-units) from linker (Vas) by solid phase chemistry. (Vas) was first coupled to the 5'-hydroxy group of the immobilized starter nucleotide, or a preassembled oligonucleotide strand, respectively. The immobilized linker (2) was detritylated to enable the synthesis of the first arm. A standard protocol for phosphoramidite chemistry was employed in all n coupling, capping and detritylation steps, except for the first three couplings that required prolonged reaction times and a three fold replenishment of the amidites. The 5'-hydroxy group of the first arm was detritylated and subsequently capped as its acetate (3). Synthesis of the second arm was achieved by palladium-catalysed removal of the Allyloxycarbonyl (Aloc) group in (3), coupling a second branching unit (Vas), and then building up the second sequence as sketched for the first one to arrive at the bisoligonucleotide (4). Following another Palladium-catalysed deprotection and the subsequent synthesis of the third arm the support was treated with ammonia to release the fully deprotected trisoligonucleotides (5), which were purified by preparative polyacrylamide gel electrophoresis under denaturating conditions, desalted by solid phase extraction and characterized by MALDI-MS.
The trisoligos synthesized are listed in Table 1a,b.

**Table 1a.**

| Constitution and selected properties of trisoligonucleotidyls (5). | | | | | | | |
|---|---|---|---|---|---|---|---|
| trisoligo^{(a)} | seq. I^{(b)} | seq. II^{(b)} | seq. III^{(b)} | yield/ nmol^{(c)} | M_{calc}^{(d)} | Mₑₓₚ^{(e)} | t_{R}/min^{(f)} |
| **A** | 5 | 7 | 12 | 107 | 14480 | 14486 | 25.05 |
| **A'** | | | | 47 | 18855 | 18839 | 25.56 |
| **A"** | | | | 16 | 18776 | 18780 | 25.33 |
| **B** | 3 | 9 | 6 | 131 | 14584 | 14587 | 25.24 |
| **B'** | | | | 42 | 18957 | 18950 | 25.59 |
| **B"** | | | | 9 | 18878 | 18917 | 25.63 |
| **C** | 11 | 2 | 4 | 61 | 14330 | 14373 | 26.31 |
| **C'** | | | | 60 | 18704 | 18697 | 25.68 |
| **C"** | | | | 17 | 18625 | 18620 | 25.60 |
| **D** | 8 | 10 | 1 | 60 | 14572 | 14575 | 25.53 |
| **D'** | | | | 53 | 18948 | 18942 | 25.87 |
| **D"** | | | | 10 | 18868 | 18773 | 25.46 |

| | | | | | | | |
|---|---|---|---|---|---|---|---|
| (a) Trisoligos A, B, C, D bear deoxycytidine as the starter base. A', B', C', D' and A", B", C", D" are connected to the 5'-end of the sequences 13 and 15 of Table 1b, respectively. | | | | | | | |
| (b) Cf. Table 1b for sequence numbering; I, II, and III denote the order by which the arms are synthesized in accordance to scheme 2. | | | | | | | |
| (c) Yield after purification from a 1.3 µmol scale; the materials were purified by denaturing PAGE (12-16% acrylamide), followed by desalting on NAP-columns (APB). | | | | | | | |
| (d) Calculated mass in Dalton. | | | | | | | |
| (e) Experimental mass as determined on a Perseptive Voyager RP/DE MALDI instrument using 3-hydroxypicolinic acid as matrix and acetonitril-H₂O (1:1) v/v for its crystallisation. | | | | | | | |
| (f) retention time during HPLC on Lichrocart C-18 (Merck), 0.2 M triethylammonium acetate, 2% acetonitrile, pH 7, as eluent A; acetonitril as eluent B. Gradient: 5 min A, 0 to 30% B within 45 min, 30 to 50% B within 1 min. | | | | | | | |

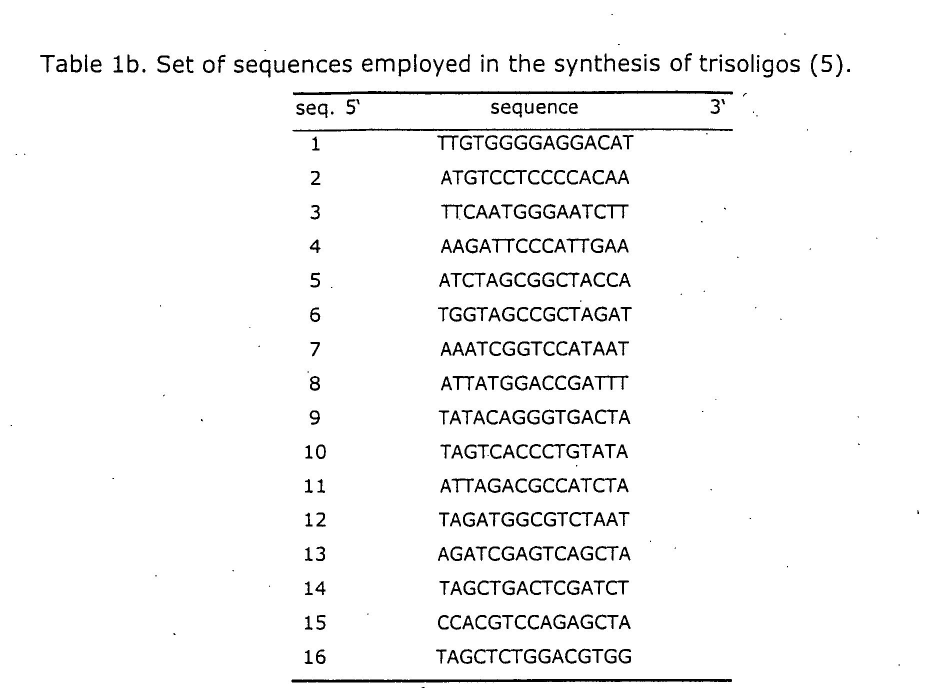

Additionally, sequences 1-12 of Table 1b were synthesized as standard oligodeoxynucleotides, while sequences 14 and 16 were modified using the 3'-terminal fluorescent dyes ROX and FAM, respectively. The set of 4 x 3 sequences for the trisoligo arms were designed to avoid mismatches, self-dimers, hairpins or loops by the nearest neighbour method described by Breslauer (Breslauer, K. J. et al., Proc. Natl. Acad. Sci. USA, 83: 3746 (1986)). Non-denaturating polyacrylamide gel electrophoresis (PAGE) of various mixtures of the linear sequences 1-12 confirmed that only complementary pairs lead to bandshifts (Example 3).

The above-mentioned templates were - besides their use for CCC - utilized for preparing tetrahedral nanoscaffold as a target for noncovalent synthesis (Figure 6).

The self assembly of trisoligo nanoobjects was studied by native gel electrophoresis. An initial screening was undertaken to find conditions for the best observability of bands with respect to the concentration of the trisoligos, the salts and buffer added, the pH, and the hybridisation protocol. Figures 7a,b show the results from a noncovalent synthesis under thermodynamic control; fast cooling as well as a variation of the trisoligo total concentration between 2.5 and 20 µM did not change the basic product pattern here. Simple mixing however without heating and cooling at all lead to a significantly higher yield of high molecular weight assemblies, whose participation could be shown by gel permeation chromatography (results not shown). From the trisoligonucleotidyls A,B,C,D all possible combinations were prepared, viz. six binary mixtures, four ternary mixtures and a single quaternary mixture. Figure 7a indicates that the products from the binary mixtures all move similarly and faster than the products from the ternary mixtures which again behave similarly on the gel. Products from the binary mixtures show the band of monomeric trisoligos, pointing to an incomplete pairing. For the case of ternary combinations, such faster moving weak bands are also visible, but to a lesser extent. For the quarternary combination however, these bands are absent indicating that pairing is complete here and pointing to a cooperative binding situation. The observed relative mobilities of 1:0.85:0.78:0.74 seen in Figure 7a are certainly more compatible with the assumption of monomers and discrete dimeric to tetrameric complexes than with higher aggregates for the case of ternary and quarternary mixtures. Similar results were obtained for trisoligonucleotidyls having presequences attached by their 5'-ends (Figure 7b). The results in Figure 7a can be rationalized as follows: Binary, ternary and quaternary mixtures lead to products where each trisoligo pairs with one, two or three arms, respectively. Following the previously introduced nomenclature suggestion, dimers have the topology of ethane-1,1,2,2-tetrayl radicals (4 unpaired arms), trimers have cyclopropyl-1,2,3-triyl topology and tetramers are fully paired having tetrahedrane topology. Complete pairing of all strands in the quartermolecular complex is further supported by digestion studies with mung bean nuclease, an enzyme with specifity for single stranded, unpaired DNA (Kowalski, D. et al., Biochemistry 15: 4457-5563 (1976); McCutchan, T. F. et al., Science 225: 626-628 (1994)). Monomeric trisoligos are digested within 5 hours (Figure 7c) while the tetrahedron ABCD is stable against digestion even after 70 hours (Figure 7d). Furthermore, when the complex A'B'C'D' bearing 4 unpaired arms with the same presequence is digested, a product is formed whose mobility is very similar to that of ABCD indicating a nearly complete removal of the presequence (Figure 7e). Contrary, if the presequence of A'B'C'D' is saturated by hybridisation with the complementary oligonucleotide, virtually no digestion takes place (Figure 7e).
Figure 8 shows the UV-melting curve from an equimolar solution of all 12 linear oligonucleotides as well as from an equimolar solution of all 4 trisoligos at similar optical densities. Additional melting curves were measured for all six individual duplexes from the respective complementary sequences of Table 1b (see Example 5). The slope of the curve for the trisoligo nanoobject (T_{M} = 52.6 °C) is steeper than in the case of the linear oligonucleotides (T_{M} = 50.4 °C) indicating that the melting of the former occurs more cooperatively. Surprisingly however, the increase of the melting temperature is only ΔT = 2.2 K. In order to gain further insight into the melting curves, thermodynamic expressions for the melting of a complex assembled from n building blocks each having β arms were derived.

Evidence for the tetrahedral geometry of the quartermolecular nanoobject came from FRET (fluorescence-resonance-energy-transfer) experiments. Five of 12 possible complexes each having the topology of 1,2-dimethyltetrahedrane were assembled from sets of trisoligos each composed of two [3+1]-arm-building blocks and two 3-arm-building blocks. The noncovalent products were purified by native gel electrophoresis, extracted from the gel and adjusted to the same concentration. The 3'-presequences allowed hybridisation with linear oligonucleotides labelled at their 3'-ends with fluorescence dyes. Figure 9 illustrates the basic structure of such assemblies. We selected fluoresceine (FAM) as the donor and rhodamine X (ROX) as the acceptor. The Förster radius given for this FRET pair is 6.0 nm (Parkhurst, M., Parkhurst, L. J., Biochemistry 34: 293 (1995)) which is close to the expected distance between the fluorophores in our assemblies, if one assumes a length of 5.1 nm for a 15mer double-stranded edge and some extra length for the linkers involved. For technical reasons, distance probing was performed on two different instruments (Table 3).

**Table 3.**

| Fluorescence readout for tetrahedral complexes hybridised with 14-ROX^{3'} and 16-FAM^{3'}. | | |
|---|---|---|
| | Readout-1 | Readout-2 |
| Complex | λₑₓᵢ = 495 nm | λₑₓᵢ = 495 nm |
| | λₑₘᵢ = 615 nm | λₑₘᵢ = 615 nm |
| A'BCD" | 84.1± 0.6 | |
| AB'CD" | 84.4± 0.4 | |
| ABC'D" | 84.3± 0.6 | 4770± 65 |
| A"B'CD | | 4822± 95 |
| A"BC'D | | 4792± 67 |
| background | 78.3± 0.5 | 6426± 51 |

Averages from 8 measurements. Readout-1: 147 nM purified complex (omitted in background measurements), 1.33 µM 14-ROX^{3'}, 1.33 µM 16-FAM^{3'} after 12 h standing at 5°C in standard TBE buffer; measured with a Kontron SFM 25 spectrofluorimeter at T = 15.4 °C. Readout-2: 93.5 nM complex, 116 nM 14-ROX^{3'}, 116 nM 16-FAM^{3'} measured with a Bio-Rad Fluoromark microplate fluorimeter at T = 15 °C. Fluorescent oligonucleotides were purchased from MWG Biotech.

Readout-1 shows the increase of the fluorescence signal of the acceptor ROX, while Readout-2 gives the decrease of fluorescence observed for the donor FAM. Ideally, the raise of the signals in Readout-1 compared to the background fluorescence should be around 40%. The increase of 8% found instead was caused by the excess of the fluorescent oligonucleotides and therefore a higher background fluorescence. In Readout-2 the concentrations of the components are more closely matched to each other. In any case, the FRET data of both readouts are similar enough to conclude that we are dealing with similar distances between the fluorophores as to be expected for a tetrahedral geometry.
We have shown that 3'-trisoligonucleotidyls with 3 different sequences including a 3'-presequence can be synthesized and utilized to construct a tetrahedral nanostructure by noncovalent assembly. The tetrahedral nanostructure is the smallest conceivable example of a spherically closed and maximally instructed object with stiff tensegrity. By attaching fluorophores to the presequences we have demonstrated the usage of stiff objects as nanoscaffolds for a spatially defined presentation of functional units. It is very likely that other functional units, such as peptides, glycosides and inorganic materials can also be attached either covalently or noncovalently to such nanoscaffolds. In an independant paper we show that unsymmetrical 3'-trisoligos can be copied into 5'-trisoligos by chemical methods (se also Eckardt, L. H. et al., submitted). If the reverse copying of 5'-linked trisoligos is also possible and will reproduce the original 3'-trisoligos, the SPREAD procedure (Luther, A., Nature 396: 245-248 (1998)) or an electronic chip variant of the latter (von Kiedrowski, G., Noxxon AG, DE 198 54 946 C2) should allow for their surface-promoted replication and exponential amplification. We therefore dare to predict that molecular robots (von Kiedrowski, G., Orig. Life Evol. Biosph. 16: 468 (1986)) and nanobots (which we understand as threedimensionally defined and multifunctionalized nanoscaffolds used as catalytic or binding machinery that can be instructed to reproduce by remote control) are not anymore a vision of the distant future (Drexler, E., Engines of Creation, Anchor press (1986); Winkless, N., Browning, I., Robots on Your Doorstep, Robotics Press, Portland (1978)) but a conceptual framework whose implementation waits on the doorstep.

### Definitions and Abbreviations

The term "template" refers to a molecule having oligonucleotide segments to which linear oligonucleotides can hybridise and are preorganised in a defined manner so that said linear oligonucleotides are coupled to each other or to a junction in a defined number, combination and orientation resulting in a defined building block. The configuration of the carbon atoms at the branching points of the template is *R* or *S*, preferably is *S*.

| | |
|---|---|
| CCC | chemical connectivity copying |
| DMAP | *N,N*-dimethylaminopyridine |
| FRET | fluorescence-resonance-energy-transfer |
| h | height |
| PAGE | polyacrylamide gel electrophoresis |
| PCR | polymerase chain reaction |
| SELEX | systematic evolution of ligands by exponential enrichment |
| SPREAD | surface-promoted replication and exponential amplification of DNA analogues |

The invention is described in more detail in the following examples which are, however, not to be construed as to limit the invention.

### Examples

### Example 1: Synthesis of the linker amidite Vas 8

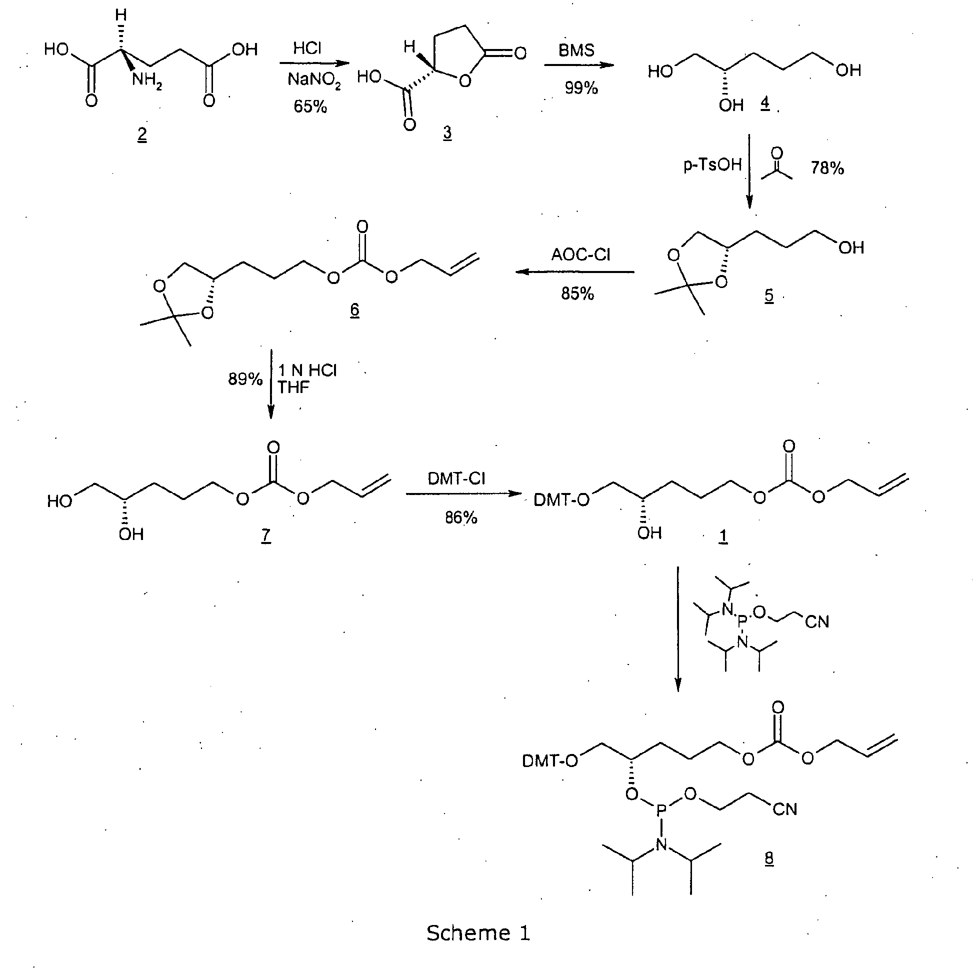

For the construction of trisoligonucleotidyls with three different sequences a linker molecule is required, which holds at least two orthogonal protecting groups. We selected 4,4'-dimethoxytrityl (DMT) and allyloxycarbonyl (AOC) for the target linker amidite. The advantage of DMT is its routine application in DNA synthesis protocols. There are of course several alternatives for the second protective group (Jarowicki, K., Kocienski, P., Contemporay Organic Synthesis 2: 315 (1995); Jarowicki, K., Kocienski, P., Contemporay Organic Synthesis 3: 397 (1996); Greene, T.W., Wuts, P.G.M., Protective Groups in Organic Synthesis, John Wiley & Sons, New York (1999); Kunz, H., Waldmann, H. in Trost, B. M., Fleming, I. (Eds.): Comprehensive Organic Synthesis, Vol. 6, p. 631, Pergamon, Oxford (1991)) for example the nitroveratryloxycarbonyl protecting group (NVOC), which we investigated earlier within this project (Dorenbeck, A., Diplomarbeit, Synthese neuer trifunktioneller Linker und Trisoligonucleotidyle, Ruhr-Universität-Bochum, Bochum (1997)). The NVOC-group is a photolabile protecting group, which can be removed by light of the wavelength λ>300 nm. The disadvantage of a photolabile protecting group is its integration in the automatised DNA-synthesis, because significant rebuildings at the DNA-synthesizer as well as a suitable laser were required. Nevertheless, first successful steps towards integration based on a quartz holder and a conventional photon source had been already realised in our lab (Dorenbeck, A., Diplomarbeit, Synthese neuer trifunktioneller Linker und Trisoligonucleotidyle, Ruhr-Universität-Bochum, Bochum (1997)). We switched to AOC protection, however, as its compatibility with automated DNA-synthesis did not call for extensive instrument modification (Scheffler, M., Dissertation, Trisoligonucleotidyle: Bausteine für ein sequenzadressiertes "self-assembly" von supramolekularen Nanostrukturen, Ruhr-Universität-Bochum, Bochum (1999)).
L-glutamic acid 2 (compound designating numbers within Example 1 refer to the numbers and corresponding structures as shown in Scheme 1) was diazotated with sodium nitrite in 2 N hydrochloric acid (Herdeis, C., Synthesis, 232 (1986)). The lactone 3 is formed by 2 successive, intramolecular, nucleophilic attacks of the containing carboxyl groups (α-lactone → γ-lactone) and therewith under complete retention (Smith, L.R., Williams, H.J., J. Chem. Edu. 56: 696 (1979)). In the second step the lactone 3 was reduced by BMS (Hornemann, A.M., Lundt, I., Tetrahedron 53: 6879 (1997)). Through the change of the reduction agent from LiAlH₄ (Veith, H.J: et al., Liebigs Ann. 2: 391 (1997) to BMS the yield could be increased from 70 to 99% due to the ease of workup. In order to introduce the first alcohol protecting group into pentane-1,2(S),5-triol 4, the vicinal diol moiety needed transient protection. Triol 4 was treated with acetone and catalytic amounts of p-toluenesulfonic acid to yield 3-[2,2-dimethyl-1,3-dioxolan-4(S)-yl]-propanol 5 in 78% yield. The AOC group was introduced by treating 5 under Schotten-Baumann-conditions with 1.2 eq allyloxycarbonyl chloride in THF in the presence of 1.2 eq. pyridine to give allyl-3-[2,2-dimethyl-1,3-dioxolan-4(S)-yl]-propylcarbonate 6 in 85 % yield. The "transient" isopropylidene group was deprotected with a mixture of 1 N HCl and THF (Angyal, S.J., Beveridge, R.J., Carbohydr. Res. 65: 229 (1978). The primary hydroxy group of the resulting ester 7 was selectively etherified with dimethoxytrityl chloride to give linker 1 in 86% yield after chromatography on silica. Its ¹H-NMR-spectra (Fig. 10) shows very clearly the splitting of diastereotopic protons, because of the chirality centre at position 4. This is especially obvious for the AB-part of an ABX-system for the protons at C-5. Some cyclohexane (1.41 ppm) is still visible although the material was dried under high vacuum. The target phosphoramidite 8 was obtained in 89% yield by esterification of 1 with 2-(cyanoethoxy)bis-N,N-(diisopropylamino)phosphane in the presence of diisopropylammonium tetrazolide.

5-Oxotetrahydrofurane-2(S)-carboxylic acid 3 (Herdeis, C., Synthesis, 232 (1986)):
2 N hydrochloric acid (210 ml) was added while stirring to 50 g (0.34 mol) L-glutamic acid 2 in 300 ml water. To the resulting solution 28.2 g (0.41 mol) sodium nitrite in 210 ml water was added dropwise within 3 h while stirring and keeping the temperature at 0°C. The mixture was stirred overnight at room temperature, evaporated to dryness and the product was extracted by stirring for 1h with a suspension of sodium sulfate in ethyl acetate. The filtrate was evaporated under reduced pressure to yield a clear yellow oil, which was dried under high vacuum. Yield: 28.65 g (≈ 65 % of th.).
¹H-NMR (200 MHz, DMSO-d₆, TMS): δ = 2.30 (m_{c}, 1H, -CH₂-), 2.62 (m_{c}, 3H, -CH₂-), 5.09 ppm (m_{c}, 1 H -OCH-COOH).
¹³C-NMR (50.3 MHz, DMSO-d₆, TMS): δ = 25.74 (C-3), 27.05 (C-4), 75.76 (C-2), 171.96 (C-5), 177.15 ppm (COOH).
MS (FAB): m/z (%) = 131 (100) [M⁺]; 107 (20); 85 (49).
C₅H₆O₄ (M = 130.10 g/mol).

Pentane-1,2(S),5-triol 4 (Hornemann, A.M., Lundt, I., Tetrahedron 53: 6879 (1997)):
BMS (10M, 8.05ml, 8.05 mmol) in 100 ml THF_{abs.} was added dropwise at room temperature to 4.86 g (37 mmol) 5-oxotetrahydrofurane-2(S)-carboxylic acid 3 in 200 ml dry THF. The solution was stirred for 3h under an argon atmosphere leading to the precipitation of a colourless solid. The reaction mixture was refluxed for 3 h at reflux, cooled to room temperature, carefully treated with methanol until complete dissolution of the solid, and coevaporated with methanol (5 x 50 ml). Yield: 4.48 g (≈ 99 % of th.).
¹H-NMR (400 MHz, DMSO-d₆, TMS): δ = 1.19 (m_{c} aa B-part of a ABX-system, 1H, 4-H), 1.43 (m_{c} aa A-part of a ABX-system, 1H, 4-H), 1.43 (m_{c} aa B-part of a ABX-system,1H, 3-H), 1.52 (m_{c} as A-part of a ABX-system, 1H, 3-H), 3.23 (m_{c}, 2H, 5-H), 3.34-3.40 (m_{c}, 3H, 1-H and X-part of two ABX-systems, 1H, 2-H), 4.35 (t, 2H, OH) 4.39 ppm (t, 1H,OH).
¹³C-NMR (100.6 MHz, DMSO-d₆, TMS): δ = 29.03 (C-3), 30.23 (C-4), 61.35 (C-1), 66.23 (C-5), 71.37 ppm (C-2).
MS (FAB): m/z (%) = 121 (100) [M+H⁺]; 107 (22); 89 (49); 85.1 (17).
C₅H₁₂O₃ (M = 120.15 g/mol).

3-[2,2-Dimethyl-1,3-dioxolan-4(*S*)-yl]-propanol 5:
To 4.04 g (34 mmol) (2*S*)-pentane-1,2,5-triol 4 in 80 ml dry acetone 0.4 g dry p-toluenesulfonic acid was added and the reaction mixture was stirred overnight at room temperature. The reaction was terminated by adding 1 ml triethylamine. After evaporation the residue was dissolved in tert-butylmethyl ether and the solution was filtered and evaporated to give the crude product which was purified by column chromatography (column: ⌀ 3 cm * h 20 cm; package solvent: cyclohexane/ethyl acetate 1/2; elution solvent: cyclohexane/ethyl acetate 1/2; detection 3 with cyclohexane/ethyl acetate 1/3 as solvent; product: R_{f} ≈ 0.41). Yield: 4.20 g (≈ 78 % ofth.).
¹H-NMR (400 MHz, DMSO-d₆, TMS): δ = 1.24 (s, 3H, CH₃), 1.28 (s, 3H, CH₃), 1.41-1.50 (m, 4H, -CH₂CH₂CH₂OH), 3.33-3.42 (m aa B-part of a ABX-system, 1H, 5'-H; 2H, 1-H), 3.92-4.04 (m aa A-part of a ABX-system, 1H, 5'-H; m aa as X-part of a ABX-system, 1H, 4'-H), 4.39 ppm (t, 1H, OH).
¹³C-NMR (100.6 MHz, DMSO-d₆, TMS): δ = 25.83 (C-methyl), 27.05 (C-methyl), 28.94 (C-3), 29.95 (C-2), 60.77 (C-5'), 68.88 (C-1), 75.54 (C-4'), 107.95 ppm (C-2').
C₈H₁₆O₃ (M = 160.21 g/mol).

Allyl-3-[2,2-dimethyl-1,3-dioxolan-4(*S*)-yl]propylcarbonate 6:
2.22 g (14 mmol) (3-[2,2-dimethyl-1,3-dioxolan-4(*S*)-yl]propanol 5 and 1.38 ml (17 mmol) pyridine were dissolved in 90 ml dry THF. The reaction mixture was cooled to 0 °C and within 30 min 1.9 ml (18 mmol) allyloxycarbonylchloride dissolved in 10 ml dry THF was added dropwise. The reaction mixture was stirred at room temperature for 10 h, filtered, evaporated to dryness and the product was purified by column chromatography (column: ⌀ 3 cm * h 10 cm; package solvent: cyclohexane/ethyl acetate 3/1; elution solvent: cyclohexane/ethyl acetate 3/1; detection 3 with cyclohexane/ethyl acetate 1/1 as solvent; product: R_{f} ≈ 0.56). Yield: 2.89 g (≈ 85 % of th.).
¹H-NMR (400 MHz, DMSO-d₆, TMS): δ = 1.25 (s, 3H, -CH₃), 1.30 (s, 3H, -CH₃), 1.52 (m_{c}, 2H, -CH₂-CH₂-O-), 1.61 (m_{c} aa B-part of a ABX-system, 1H, (3)-H), 1.67 (m_{c} aa A-part of ABX-system, 1H, (3)-H), 3.43 (m_{c} aa B-part of a ABX-system, 1H, [5]-H), 3.97 (m_{c} aa A-part of a ABX-system, 1H, [5]-H), 4.03 (m_{c} aa as X-part of two ABX-systems, 1H, [4]-H), 4.11 (t, ³J= 6.52 Hz, 2H, (1)-H), 4.58 (dt, ³J= 5.52 Hz, ⁵J= 1.5 Hz, 2H, CH₂-CH=CH₂), 5.27 (2dm, ³Jₜᵣₐₙₛ= 17.15 Hz, ³J_{cis}= 10.55 Hz, 2H, -CH=CH₂), 5.92 ppm (m_{c}, 1H, -CH=CH₂).
¹³C-NMR (100.6 MHz, DMSO-d₆, TMS): δ = 24.75 (C-(3)), 25.65 (C-methyl), 26.89 (C-methyl), 29.41 (-CH₂-CH₂-O-C(O)), 67.53 (-CH₂-CH₂-O-C(O)), 67.75 (CH₂-CH=CH₂), 68.61 (C-[5]), 75.04 (C-[4]), 108.04 (C-[2]), 118.27 (-C=CH₂), 132.36 (-C=CH₂), 154.47 (C=O) ppm.
C₁₂H₂₀O₅ (M = 244.29 g/mol).

Allyl-4(S),5-dihydroxypentylcarbonate 7 (Angyal, S.J., Beveridge, R.J., Carbohydr. Res., 65: 229 (1978)):
100 ml 1 N hydrochloric acid was added dropwise to 10.71 g (44 mmol) allyl-3-[2,2-dimethyl-1,3-dioxolan-4(S)-yl]propyl carbonate 6 in 100 ml THF, while stirring and cooling with an ice bath. Stirring was continued for 24 at room temperature. The solution was evaporated to dryness and the product was purified by column chromatography (column: ⌀ 5.5 cm * h 8 cm; package solvent: cyclohexane/ethyl acetate 1/3; elution solvent: cyclohexane/ethyl acetate 1/3;
detection 3 with cyclohexane/ethyl acetate 1/1 as solvent; product: R_{f} ≈ 0.22).
Yield: 7.94 g (≈ 89 % of th.).
¹H-NMR (400 MHz, DMSO-d₆, TMS): δ = 1.27 (m_{c} aa B-part of a ABX-system, 1H, 3'-H), 1.49 (m_{c} aa A- part of a ABX-system, 1H, 3'-H), 1.61 (m_{c} aa B- part of a ABX-system, 1H, 2'-H), 1.74 (m_{c} aa A- part of a ABX-system, 1H, 2'-H), 3.24 (m_{c} aa B- part of a ABX-system, 1H, 5'-H), 3.29 (m_{c} aa A- part of a ABX-system, 1H, 5'-H), 3.41 (m_{c} aa X-part of three ABX-Systems, 1H, 4'-H), 4.10 (t, ³J= 6.72 Hz, 2H, 1'-H), 4.44 (m_{c}, 2H, -OH), 4.59 (d, ³J= 5.52 Hz, 2H, CH₂-CH=CH₂), 5.29 (2d, ³Jₜᵣₐₙₛ= 17.3 Hz, ³J_{cis}= 10.46 Hz, 2H, -CH=CH₂), 5.93 ppm (ddt, ³J= 5.52 Hz, ³J_{cis}= 17.2 Hz, ³Jₜᵣₐₙₛ= 17.2 Hz, 1H, -CH=CH₂).
¹³C-NMR (100.6 MHz, DMSO-d₆, TMS): δ = 25.45 (C-2'), 30.31 (C-3'), 66.67 (C-1'), 68.46 (CH₂-CH=CH₂), 68.77 (C-5'), 71.51 (C-4'), 119.06 (CH₂-CH=CH₂), 133.13 (CH₂-CH=CH₂), 155.27 ppm (C-1).
C₉H₁₆O₅ (M = 204.22 g/mol).

Allyl-5-(4,4'-dimethoxytrityl)oxy-4(*S*)-hydroxypentyl carbonate 1:
7.90 g (39 mmol) allyl-4(*S*),5-dihydroxypentylcarbonate 7 as dissolved in a small amount of absolute pyridine. 13.17 g (0.39 mmol) dimethoxytrityl chloride, dissolved in 100 ml absolute pyridine, was added dropwise while stirring over six hours at 0 °C. After stirring for 12 h, the reaction mixture was diluted with 50 ml methanol, stirred for 30 min at room temperature and evaporated to dryness. The residue was purified by column chromatography. The product was dried under high vacuum (column: ⌀ 9 cm * h 16 cm; package solvent: cyclohexane/ethyl acetate 3/1 with 2 % triethylamine; elution solvent: cyclohexane/ethyl acetate 3/1; detection 1 with cyclohexane/ethyl acetate 3/1 as solvent; product: R_{f} ≈ 0.22).
Yield: 16.92 g (≈ 86 % of th.).
¹H-NMR (400 MHz, CDCl₃, TMS): δ = 1.47 (m_{c} aa A- and B-part of a ABX-system, 2H, 2-H), 1.66 (m aa B-part of a ABX system, 1H, 3-H), 1.79 (m aa A-part of a ABX-system, 1H, 3), 2.32 (d, ³J= 3.5 Hz, 1H, OH), 2.99-3.16 (8 signals of a AB-part of a ABX-system, J_{AB}≈ 9.5 Hz, J_{AX}≈ 2.4 Hz, J_{BX}≈ 6.7 Hz, 2H, 5-H, ν_{A}= 3.15, ν_{B}= 3.01), 3.77 (s, 6H, O-CH₃ von DMT; 1H, X-Teil of two ABX-systems, 4-H), 4.12 (m, 2H, 1-H), 4.59 (dt, ³J= 6.02 Hz, ⁵J= 1.5 Hz, 2H, CH₂-CH=CH₂), 5.22-5.35 (2*dm, ³Jₜᵣₐₙₛ= 17.07 Hz, ³J_{cis}= 10.04 Hz, 2H, -CH=CH₂), 5.90 (ddtt, ³J= 6.02 Hz, ³J_{cis}= 17.07 Hz, ³Jₜᵣₐₙₛ= 17.07 Hz, 1H, -CH=CH₂), 6.81 (d, 4H, 3,3' and 5,5' DMT H arom.), 7.17-7.41 ppm (m, 9H, remaining arom. DMT H).
¹³C-NMR (100.6 MHz, CDCl₃, TMS): δ = 24.89 (C-2), 29.49 (C-3), 55.18 (OCH₃), 67.46 (C-1), 67.96 (CH₂-CH=CH₂), 68.28 (C-4), 70.44 (C-5), 86.14 (Cq-DMT), 113.14 (C-3,3' and C-5,5' DMT), 118.79 (CH₂-CH=CH₂), 126.81 127.83 128.11 129.11 (restl. DMT C), 130.01 (C-2,2' and C-6,6' DMT), 131.63 (CH₂-CH=CH₂), 135.93 (C-1 and 1' from Ph-OCH₃ of DMT), 144.77 (C-1 from Ph of DMT), 155.00 (C=O), 158.53 (C-4 u. 4' from Ph-OCH₃ of DMT).
MS (FAB): m/z (%) = 505.2 (0.05) [M⁺]; 454.2 (0.16); 438.2 (0.2); 409.2 (0.32); 379.2 (0.19); 343.2 (0.32); 331.2 (0.43); 303.1 (100).
C₃₀H₃₄O₇ (M = 506.6 g/mol).

1-Allyloxycarbonyloxy-5-(4,4'-dimethoxytrityl)oxypent-4(*S*)-yl-O-(β-cyanoethyl-N,N-diisopropyl)phosphoamidite 8:
Under an argon atmosphere 1.62 g (5.4 mmol) 2-(cyanoethoxy)bis-N,N-(diisopropylamino)phosphane was added dropwise over 30 min to a stirred solution of 2.25 g (4.4 mmol) allyl-5-(4,4'-dimethoxytrityl)oxy-4(S)-hydroxypentylcarbonate (1) and 1 g (5.8 mmol) diisopropylammoniumtetrazolide in dry CH₂Cl₂ at 0 °C. Stirring was continued for 2 h at room temperature. The mixture was evaporated and the product was purified by column chromatography (column: ⌀ 9 cm * h 6 cm; package solvent: cyclohexane/ethyl acetate 4/1 with 2 % triethylamine; elution solvent: cyclohexane/ethyl acetate 4/1; detection 1 with cyclohexane/ethyl acetate 4/1 as solvent; product: R_{f}≈ 0.17).
Yield: 2.8 g (≈ 89 % of th.).
¹H-NMR (600 MHz, CDCl₃, TMS): δ = 1.04 (d, ³J= 6.79 Hz, 3H, CH(CH₃)₂), 1.14 (q, ³J= 6.60 Hz , 9H, CH(CH₃)₂), 1.60-1.81 (m aa 2 AB-parts of two ABX systems, 4H, 2-H u. 3-H), 2.36 u. 2.59 (t, ³J= 6.34 Hz, 2H, CH₂CN), 2.91-3.20 (m aa A- and B- part of a ABX-system, 2H, CH₂ODMT), 3.49-3.60 (m, 2H, CH(CH₃)₂), 3.60-3.85 (m aa A- and B- part of a ABX-system, 2H, CH₂OP), 3.76 and 3.77 (s, 6H, OCH₃ DMT), 3.97 (m, 1H, CH-O-P), 4.11 (m_{c}, 2H, 1-H), 4.58 (m_{c}, 1H, X-part of two ABX-systems, 4), 4.12 (m, 2H, 1-H), 4.59 (dt, ³J= 6.02 Hz, ⁵J= 1.5 Hz, 2H, CH₂-CH=CH₂), 5.22-5.35 (2*dm, ³Jₜᵣₐₙₛ= 17.2Hz, ³J_{cis}= 10.41 Hz, 2H, -CH=CH₂), 5.90 (m_{c}, 1H, -CH=CH₂), 6.79 (d, 4H, 3,3' u. 5,5' DMT H arom.), 7.15-7.43 ppm (m, 9H, remaining arom. DMT H).
¹³C-NMR (100.6 MHz, CDCl₃, TMS): δ = 23.39 (CH₂CN), 24.72 (C-2), 26.91 (CH(CH₃)₂), 28.03 (C-3), 43.12 (CH(CH₃)₂), 55.16 (OCH₃), 64.28 (CH₂OP), 68.19 (CH₂ODMT), 68.28 (C-1), 68.52 (CH₂-CH=CH₂), 71.79 (C-4), 85.75 (Cq-DMT), 112.92 (C-3,3' u. C-5,5' DMT), 118.66 (CH₂-CH=CH₂), 126.56 127.63 128.20 128.27 (restl. DMT C), 130.08 (C-2,2' u. C-6,6' DMT), 131.78 (CH₂-CH=CH₂), 135.27 (C-1 u. 1' from Ph-OCH₃ of DMT), 145.12 (C-1 from Ph of DMT), 155.00 (C=O), 158.33 (C-4 u. 4' from Ph-OCH₃ of DMT).
³¹P-NMR (162 MHz, CDCl₃, 85 %-phosphoric acid): δ = 148.07, 149.07 ppm diastereomers as result of the chiral phosphorus.
C₃₉H₅₁N₂O₈P (M = 706.82 g/mol).

### Example 2

The synthesis of the template was effected according to the general reaction scheme shown in Fig. 5 (see also Dorenbeck, thesis Unvi. Bochum (2000)).

### Example 3

### Synthesis of 3-arm 5'-linked trisolgonucleotidyls using chemical copying of connectivity

2 µl of template Y₍₄₅₎ (100 µM) wherein the 3' end of the oligonucleotides A' to C' was attached to the core structure, were added to 10 µl of reactionbuffer (100 mM MOPS; pH 5,6 ). After addition of one equivalent of each modified oligonucleotide (A-15, B-13, C-11) the solution was vortexed and heated two min at 60°C. After rapid cooling to room temperature 1 to 2.5 equivalents of the trislinker (1,3,5-Trisformyfbenzene) dissolved in DMF was added and the reactionmixture was shaked for 2 h. The formed hydrazone linkage could be reduced using 100 equivalents of NaCNBH₃. The product (39-mer) was isolated by using polyacrylamide gel electrophoresis under denaturating conditions, desalted by solid phase extraction and characterized by MALDI-MS.

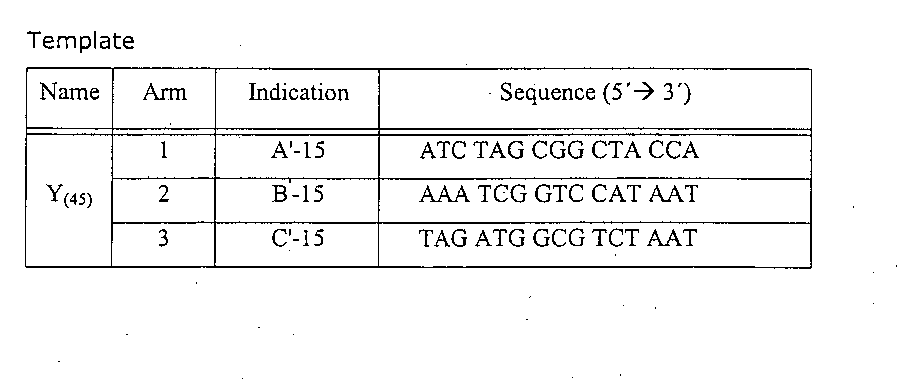

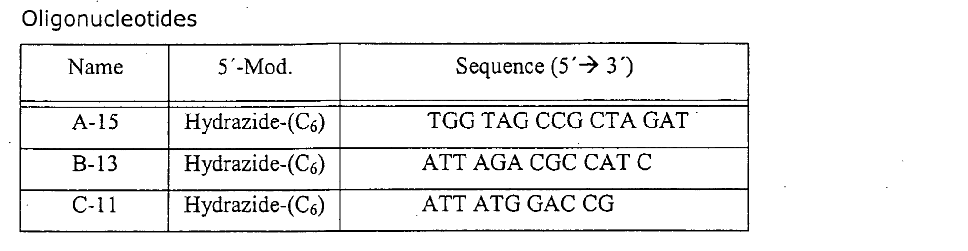

Results of electrophoresis

| | **Reactionmixture** | | | |
|---|---|---|---|---|
| **Lane** | **Template** | **Oligonucleotide** | **Trislinker (eq.)** | **pH** |
| **1** | Y₍₄₅₎ | A-15, B-11, C-13 | 1 | 5.6 |
| **2** | Y₍₄₅₎ | A-15, B-11, C-13 | 2.5 | 5.6 |
| **3** | Y₍₄₅₎ | A-15, B-11, C-13 | 1 | 5.6 |
| **4** | Y₍₄₅₎ | A-15, B-11, C-13 | 2.5 | 5.6 |
| **5** | - | A-15, B-11, C-13 | 1 | 5.6 |
| **6** | - | A-15, B-11, C-13 | 2.5 | 5.6 |

The corresponding gel is shown in Fig. 3.

### Example 4

Tris-linking with shortened templates: Similar experiments as that of Example 3 were performed with shortened templates (again the following oligonucleotides A' to C' of the templates were attached with its 3'-end to the Vas linkers). The results are shown in Fig. 10.

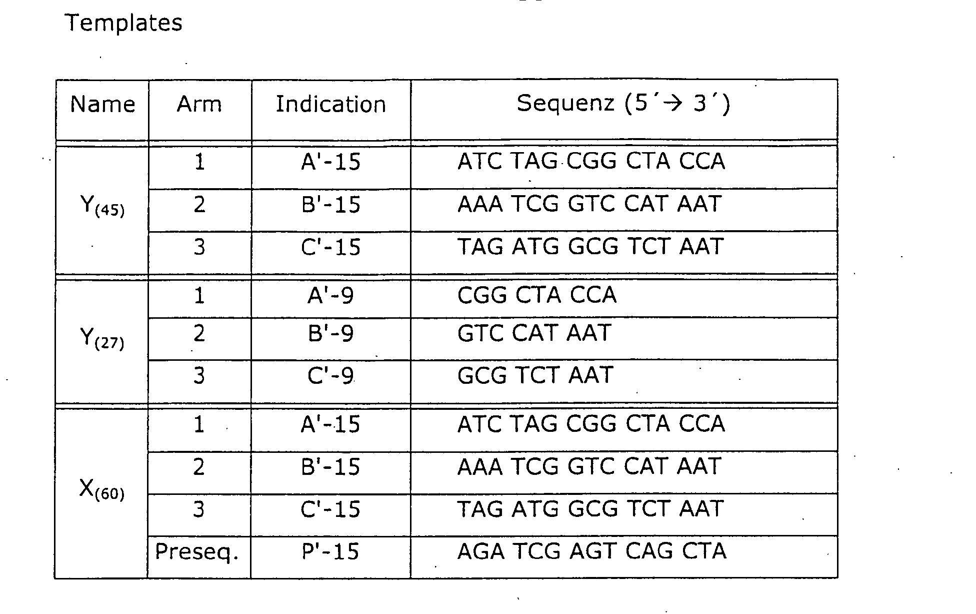

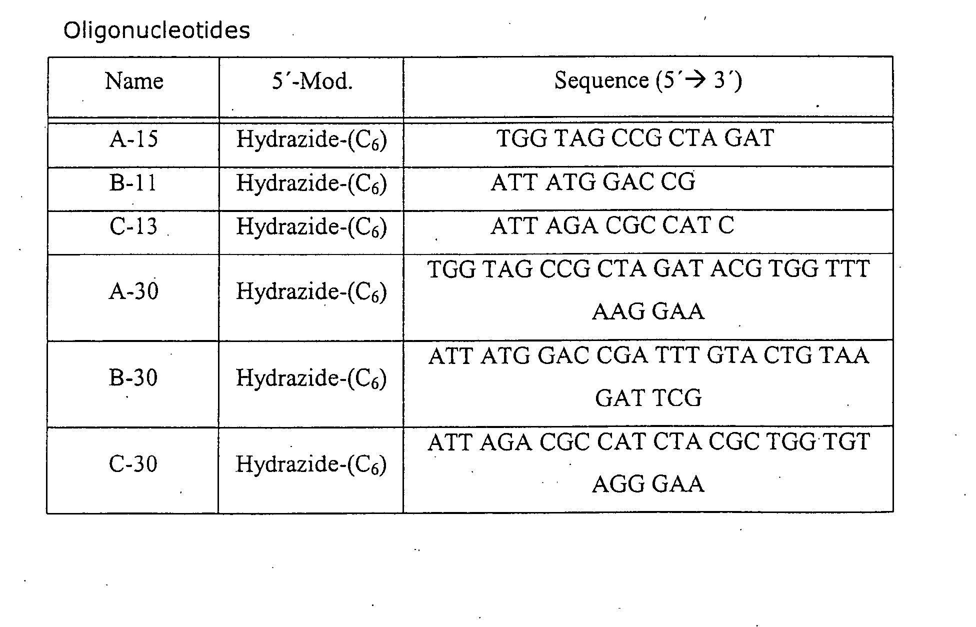

## Claims

1. Method for the chemical copying of connectivity which comprises coupling a set of linear oligonucleotide segments to a junction element in the presence of one or more branched templates having oligonucleotide arms to form branched copies of the templates.

2. The method of claim 1, wherein
(i) the molecular templates and the copies of the templates have at least three, preferably linear oligonucleotide arms/segments; and/or
(ii) said oligonucleotide segments and said oligonucleotide arms independently comprise from 3 to 1000, preferably from 5 to 100, most preferably from 9 to 30 nucleotides; and/or
(iii) the linear oligonucleotide segments of the copies of the templates have at least 3 consecutive nucleotides hybridising to at least 3 consecutive complementary nucleotides of one of the oligonucleotide arms of the template; and/or
(iv) the oligonucleotide arms of the templates have the same or different nucleotide sequence, preferably have different nucleotide sequences, and preferably are covalently bound to a core structural element; and/or
(v) the linear oligonucleotide segments of the copies of the templates are covalently coupled to a junction element having at least three functional groups which are reactive to a corresponding functional group present on each of said linear oligonucleotide segments; and/or
(vi) the junction element is capable of making covalent bonds to at least 3 oligonucleotide segments, preferably the junction element has at least three functional groups as defined under (v) above.

3. The method according to claim 1 or 2, wherein the templates have the formula (I)
L-D(S-A)ₙ (I)
wherein D is the core structural element, A represents independently the oligonucleotide arms of the template which may be independently oriented in both possible directions, S is a suitable spacer group capable of connecting the oligonucleotides arms to the core, L is an optional linker suitable for binding the template to a solid support, n is ≥ 3, preferably D is selected from a substituted or unsubstituted branched alkyl residue, a cycloalkyl residue, an aryl residue, a phosphanyl or phosphonate residue, a phosphate, an amine, a cyclopeptidyl residue and a cyclodextryl residue, S and L are functionalized linear alkyl residues which may be substituted or may carry heteroatoms and may preferably contain phosphates as functional moieties, and n is from 3 to 5, preferably is 3.

4. The method of claim 3, wherein the template corresponds to formula (II) 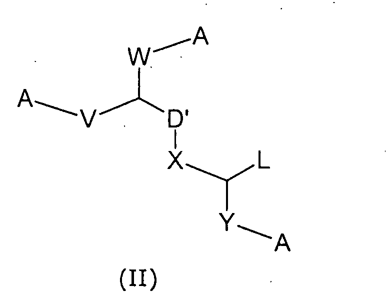 wherein A and L are as defined in claims 2 and 3, D' has the same meaning as D defined in claim 3, and V, W, X and Y are optionally substituted alkyl residues.

5. The method of claim 4, wherein the template corresponds to formula (III) 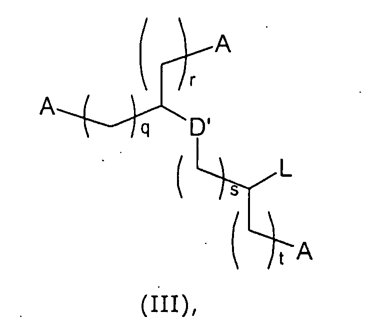 wherein A and L are as defined in claim 2, 3 or 4; D' is a phosphate, q, r, s and t are integers each ranging independently from 0 to 20, provided that r and q are not 0 at the same time, preferably q, r, s and t range from 1 to 5, more preferably q = t = 1 and r = s = 3.

6. The method according to any of claims 1 to 5, wherein the linear oligonucleotide segments A' comprise optionally a suitable spacer group S conjugated to one of its ends or to a central portion of the segment, which are bonded to a functional junction element B' so that the resulting copies of the templates correspond to the formula (IV)
B(S-A')ₙ (IV)
wherein S is a suitable spacer group as defined in claim 3, A' has the same meaning as A defined in claims 3 or 4, n has the same meaning as defined in claims 3 or 4, and B is a junction element as defined in claim 2.

7. The method of claim 6, wherein
(i) B is cyclic or heterocyclic, preferably an aromatic or heteroaromatic ring having at least 3 same or different functional groups, more preferably the ring having 3 identical functional groups, in C_{3V} symmetry said functional groups being capable of bounding to the linear oligonucleotide segments or their conjugates of formula (I) and/or (II), still more preferably being selected from one or more of - OH, -SH, -CHO, -COOH, -NH₂, -NHNH₂, -ONH₂, maleimide, or vinylsulfone and functionally equivalent groups, still more preferably being an electrophilic group, most preferably being -CHO; and/or
(ii) S is a linear alkyl residue connected to A' via phosphodiester linkage and having from 1 to 20, preferably from 1 to 6 carbon atoms, being optionally interrupted by a heteroatom and having one or more functional groups independently selected from -OH, -SH, -CHO, -COOH, -NH₂, -NHNH₂, at least one of which is reactive to a functional group of the junction element as defined under (i) above, preferably is a nucleophilic, nitrogen containing group, more preferably is -NHNH₂.

8. The method of claim 7, wherein
(i) S corresponds to formula (V) 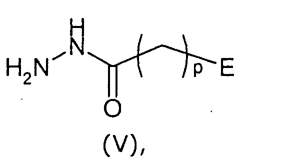 wherein
(a) p is an integer ranging from 1 to 10, preferably from 1 to 6, most preferably is 5; and
(b) E is a chemical bond, a heteroatom, a phosphate, a pyrophosphate, an amide or carbonyl bond to A'; and/or
(ii) B corresponds to formula (VI) and (VII):
N[(CH₂)₂(CH₂)ₖNHCOCHO]₃ (VI) 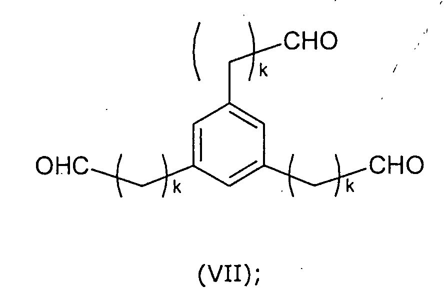
wherein k is from 0 to 2, preferably k is 0.

9. The method according to any one of claims 1 to 8 wherein the oligonucleotides segments and oligonucleotides arms are independently selected from DNA, RNA, PNA, and LNA based structures, and pRNA polymers, preferably are DNA based structures or pRNA polymers.

10. The method according to any one of claims 1 to 9, wherein
(A) the segments and arms are DNA structures and the template has the structure 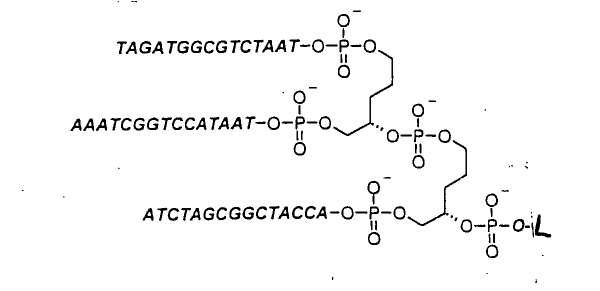 wherein L is (the remainder of) a linker to a solid support, the junction element and the three linear oligonucleotides have the following structures 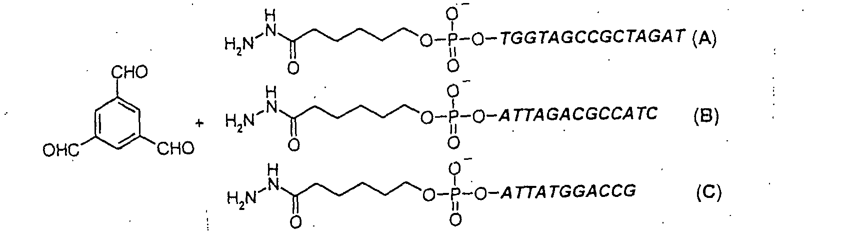 and
(B) pRNA structures corresponding to that shown in (A) above.

11. The method according to any one of claims 1 to 10
(i) wherein the copies of the templates are utilized as templates in a subsequent round of copying, and/or
(ii) the number of nucleotides within the oligonucleotides arms of the template can vary significantly, preferably a long arm can be up to ten times longer than the short arm of the template, such templates are suitable for elongating the segment within the copy of the template corresponding to the short arm.

12. A kit for chemical copying of connectivity comprising one or more of the compounds as defined in any of claims 1 to 10.

13. The copy of the template as defined in any of claims 1 to 11.
